(19) Europäisches Patentamt
European Patent Office
Office européen des brevets

(11) Veröffentlichungsnummer: **0 403 960 A2**

(12) **EUROPÄISCHE PATENTANMELDUNG**

(21) Anmeldenummer: **90111263.1**

(51) Int. Cl.5: **A61K 47/48, C07D 213/52, C07C 321/08**

(22) Anmeldetag: **14.06.90**

(30) Priorität: **19.06.89 DE 3919923**

(43) Veröffentlichungstag der Anmeldung: **27.12.90 Patentblatt 90/52**

(84) Benannte Vertragsstaaten: **AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Hermentin, Peter, Dr.**
**Salzköppel 9**
**D-3550 Marburg(DE)**
Erfinder: **Dönges, Reiner**
**Schöne Aussicht 1d**
**D-3554 Gladenbach(DE)**
Erfinder: **Franssen, Udo, Dr.**
**Salzköppel 11**
**D-3550 Marburg(DE)**
Erfinder: **Enssle, Karlheinz, Dr.**
**Salegrund 4**
**D-3550 Marburg(DE)**
Erfinder: **Kurrle, Roland, Dr.**
**Schenkendorfweg 18**
**D-3550 Marburg(DE)**
Erfinder: **Seiler, Friedrich-Robert, Dr.**
**Oberer Eichweg 10**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Magnetische Protein-Konjugate, Verfahren zu ihrer Herstellung und ihre Verwendung.**

(57) Die Erfindung betrifft magnetische Protein-Konjugate der allgemeinen Formel I,

$M-NH-CO-(CH_2)_n-S-SP$ I

mit n = 1-6, bevorzugt mit n = 2 oder 3, worin M ein dispergierbares, magnetisch reagierendes Material oder Partikel, welches Aminogruppen trägt, und P ein Protein ist, ein Verfahren zu ihrer Herstellung sowie ihre Verwendung zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten oder im Rahmen eines diagnostischen Verfahrens bzw. als Diagnostikum, bevorzugt zur Entfernung von Zellen, bevorzugt zur Knochenmark-Depletion oder zur HLA-Typisierung.

EP 0 403 960 A2

## Magnetische Protein-Konjugate, Verfahren zu ihrer Herstellung und ihre Verwendung

Die Erfindung betrifft magnetische Protein-Konjugate der Formel I,

$M-NH-CO-(CH_2)_n-S-S-P$ I

nit n = 1-6, bevorzugt mit n = 2 oder 3, für welche gilt:

M ist ein dispergierbares, magnetisch reagierendes Material oder Partikel, welches Aminogruppen trägt, und P ist ein Protein.

P kann ein Protein sein, in welchem die für die Bindung in I nötige Thiolgruppe entweder in natürlicher Weise vorhanden ist oder durch Reduktion von Disulfidbindungen erzeugt oder durch chemische Reaktion eingeführt wird.

P ist insbesondere ein Immunglobulin oder Immunglobulin-Rest, bevorzugt ein monoklonaler Antikörper oder ein Fab-, Fab'- oder $F(ab')_2$- Fragment, ein Antigen oder ein Enzym-, Hormon-, Lectin- oder Wachstumsfaktorrest.

P ist bevorzugt ein monoklonaler Antikörper der IgG- oder IgM-Klasse, insbesondere ein monoklonaler Antikörper, welcher gegen ein Antigen gerichtet ist, welches in wäßrigen Salzlösungen oder Körperflüssigkeiten in gelöster Form vorhanden ist oder ein monoklonaler Antikörper, welcher gegen ein Antigen gerichtet ist, welches auf Zellen exprimiert ist, wobei die das Antigen exprimierenden Zellen insbesondere Zellen des myeloischen oder lymphatischen Systems, Zellen des peripheren Bluts, insbesondere B-Lymphozyten, T- Lymphozyten oder deren Vorläuferzellen oder Tumorzellen, insbesondere Tumorzellen des Knochenmarks sein können. Solche Zellen können auch Erythrozyten, Bakterien, Mykoplasmen oder Protozoen sein. Im Rahmen der Erfindung sollen aber auch Viren als Zellen verstanden werden.

M ist bevorzugt ein dispergierbares Partikel mit Metalloxid-Core und einem Aminogruppen tragenden Hüllmantel, wobei das Metalloxid-Core eine Gruppe paramagnetischer Substanzen einschließen kann, bevorzugt ein Partikel dessen Durchmesser zwischen etwa 0,1 $\mu$ und etwa 100 $\mu$, bevorzugt jedoch zwischen etwa 0,1 $\mu$ und etwa 1,5 $\mu$ liegt.

Die Erfindung betrifft ferner ein Verfahren zur Herstellung eines magnetischen Protein-Konjugats der allgemeinen Formel I sowie die Verwendung eines Konjugats der Formel I zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten sowie die Verwendung im Rahmen eines Diagnostikums bzw. als Diagnostikum sowie insbesondere die Verwendung zur Knochenmarks-Depletion oder zur HLA-Typisierung.

Eine Knochenmarkstransplantation ist unter anderem bei der Behandlung bestimmter Formen der Leukämie und der Panmyelopathie (Knochenmarksschwund) oftmals die einzige Möglichkeit der Therapie.

Bei Leukämien und gewissen lymphoiden Neoplasien werden die Patienten bisweilen einer Ganzlkörperbestrahlung mit extrem hoher Dosis und/oder einer aggressiven Chemotherapie unterworfen. Bei einer derartigen Behandlung werden die normalen Stammzellen des Knochenmarks, die Vorläufer aller Blutzellen, vollständig zerstört. Dem Patienten wird daher Knochenmark eines geeigneten Spenders reinfundiert, von dem sich Zellen in den Knochenmarkräumen des Empfängers ansiedeln und so die Neuentwicklung des Blutbildungs-und Immunsystems ermöglichen. Dieses Verfahren nennt man allogene Knochenmarkstransplantation.

Verantwortlich für das hohe Risiko der allogenen Knochenmarkstransplantation sind unter anderem die dem Patienten im reinfundierten Knochenmark mitübertragenen T-Lymphozyten des Spenders, welche die Zellen des Empfängers als fremd erkennen und sie deshalb angreifen und zerstören. Diese für den Patienten oft lebensbedrohende Knochenmarksunverträglichkeit nennt man "graft-versus-host"-Reaktion oder "graft-versus-host"-Erkrankung (GVHD). Die Risiken dieser "graft-versus-host"-Erkrankung können zum einen dadurch vermindert werden, daß man dem Patienten nach Möglichkeit genau typisiertes Knochenmark besonders geeigneter Spender, meist aus dem Verwandtenkreis, reinfundiert. Zum anderen können sie aber auch dadurch reduziert werden, daß man unerwünschte Zellpopulationen, wie sie z. B. T-Lymphozyten des Spender-Knochenmarks darstellen können, vor der Reinfusion in den Patienten selektiv eliminiert. Diese Elimination von Spender-T-Zellen kann z. B. durch selektive Lysis der zu entfernenden Zellen in Gegenwart von Komplement oder durch selektive Abtötung der T-Zellen mit Hilfe sogenannter Inmuntoxine oder durch ein anderes Verfahren, z. B. durch magnetische Zell-Depletion des Knochenmarks erfolgen.

Eine derartige Zell-Depletion von Knochenmark kann man auf relativ einfache Weise so durchführen, daß man das Knochenmark mit einem monoklonalen Antikörper von der Maus inkubiert, welcher z. B. spezifisch gegen die T-Zellen des Knochenmarks gerichtet ist und sich infolgedessen nur an die T-Zellen

bindet. Derartige, mit monoklonalen Antikörpern von der Maus belegte T-Zellen können nun in einem zweiten Schritt dadurch entfernt werden, daß man sie z. B. mit anti-Maus-Immunglobulin vom Kaninchen, welches an magnetische Partikel gebunden ist, inkubiert, wodurch die T-Lymphozyten in spezifischer Weise mit dem magnetischen Material belegt werden, so daß sie sich mit Hilfe eines Magneten aus dem Knochenmark entfernen lassen (s. hierzu Vartdal et al., Transplantation (1987), 43, 366-371 und die dort zitierte Literatur).

In analoger Weise lassen sich aus dem Knochenmark auch andere Zellpopulationen, etwa Tumorzellen, entfernen, was für die sogenannte autologe Knochenmarkstransplantation von Bedeutung ist (s. hierzu Kvalheim et al., Cancer Research (1987), 47, 846-851 und die dort zitierte Literatur). Dabei kann, wie von Kvalheim et al., ibid., beschrieben, der die Tumorzellen erkennende monoklonale Antikörper auch direkt an die magnetischen Partikel gebunden werden, so daß der oben erwähnte Zweitantikörper (Kaninchen anti-Maus) nicht mehr benötigt wird.

Das vorstehend beschriebene Verfahren zur Knochenmarks-Depletion mit Hilfe monoklonaler oder polyklonaler Antikörper, welche an magnetische Partikel gebunden sind, ist noch sehr jung und bedarf der weiteren Entwicklung und Erprobung. Hierfür geeignete magnetische Partikel stehen heute in verschiedenster Form käuflich zur Verfügung, und ihre Herstellung wurde mehrfach in der Patentliteratur beschrieben (s. z. B. Chagnon et al., EP 0125995 A2 (Priorität US 493991 vom 12.05.83), Advanced Magnetics, oder Ughelstad et al., WO 8303920 vom 10.11.83, SINTEF). Von diesen magnetischen Partikeln ist bekannt, daß sie aus einem Metalloxid-Core bestehen, in welchem paramagnetische Substanzen eingeschlossen sein können, und daß das Core von einem Hüllmantel umgeben ist, welcher reaktive Gruppen, wie z. B. Aminophenyl-, Amino-, Carboxyl-, Hydroxy-oder Sulfhydrylgruppen, tragen kann, welche zur Kopplung von Proteinen verwendet werden können (Chagnon et al., EP 0125995 A2).

Bekannt ist beispielsweise, daß Carboxylgruppen tragende Partikel in Gegenwart eines Kondensationsmittels mit Aminogruppen von Proteinen zur Reaktion gebracht werden können (Chagnon et al., EP 0125995 A2).

Bekannt ist ferner die Kopplung von Proteinen an Aminogruppen tragende magnetische Partikel unter Verwendung von Glutaraldehyd, wobei die Kopplung jeweils über die Aminogruppen erfolgt (Chagnon et al., EP 0125995 A2).

Bekannt ist weiterhin, daß sich Hydroxygruppen tragende Partikel durch Reaktion mit p-Toluolsulfonylchlorid aktivieren lassen, und daß solchermaßen aktivierte Partikel mit Aminogruppen von Proteinen zur Reaktion gebracht werden können (Kvalheim et al., Cancer Research (1987), 47, 846-851).

All diese Kopplungsverfahren haben gemeinsam, daß das Protein jeweils über seine freien Aminogruppen an die Partikel angeknüpft wird. Eine derartige Kopplung über Aminogruppen kann jedoch bei monoklonalen Antikörpern von erheblichem Nachteil sein, weil hierbei bisweilen die Spezifität und Reaktivität der Antikörper beeinträchtigt wird. Dies ist eine Folge der Tatsache, daß sich die Aminogruppen bei einem Antikörper sozusagen statistisch über das ganze Molekül verteilen und daher auch in der Antigen-Bindungsstelle der Fab-Fragmente lokalisiert sind, was bei einer Kopplung über diese Aminogruppen einen Spezifitätsverlust bewirkt.

Es ist weiterhin bekannt, daß sich Antikörper auch ohne chemische Verknüpfung rein adsorptiv auf magnetische Partikel aufziehen lassen, wenn die Partikel aus einem Styrol-Divinylbenzol-Copolymerisat bestehen, welches Eisenoxid enthält, da sich Protein bekanntlich unspezifisch an Polystyrol anbindet.

Auch bei diesem Verfahren muß jedoch mit einer Beeinträchtigung der Antikörperspezifität und -reaktivität gerechnet werden. Ein weiterer gravierender Nachteil dieses Verfahrens besteht jedoch darin, daß sich adsorptiv gebundene Antikörper bei der Knochenmarks-Depletion wieder ablösen und so dem Patienten bei der Reinfusion des depletierten Knochenmarks mit verabreicht werden können, was insbesondere bei einem vorangegangenen Therapieversuch mit monoklonalen Antikörpern zu ernsten Nebenreaktionen führen könnte. Dieses Problem ist jedoch bekannt und soll durch kovalente Anknüpfung der Antikörper an die magnetischen Partikel überkommen werden.

Bekannt ist auch, daß magnetische Partikel auf Polystyrol-Basis den gravierenden Nachteil haben, daß sie zu Aggregation neigen und sich an Zellen auch unspezifisch anlagern.

Ausgehend von diesem Stand der Technik ist es die Aufgabe der vorliegenden Erfindung, eine Methode zu entwickeln, bei welcher monoklonale Antikörper an magnetische Partikel a) kovalent und b) nicht über ihre Aminogruppen gekoppelt werden. Aufgabe der vorliegenden Erfindung ist es daher, mit anderen Worten, ein Kopplungsverfahren zu finden, bei welchem die Antigen-Bindungsstelle des Antikörpers nicht verändert wird bzw. die Kopplung des Antikörpers abseits der Antigen-Bindungsstelle erfolgt.

Gelöst wird diese erfindungsgemäße Aufgabe durch Herstellung magnetischer Protein-Konjugate der Formel I.

Es wurde bereits vorgeschlagen, Aminogruppen tragende magnetische Partikel in magnetische Partikel

zu überführen, welche als reaktive Gruppen Maleimido-Funktionen tragen und diese mit Proteinen zu konjugieren, welche Thiolgruppen besitzen, wobei die Thiolgruppen im Protein entweder bereits nativ vorhanden oder auf chemischem Wege eingeführt oder durch Reduktion vorhandener Disulfidbindungen erzeugt werden können.

Es wurde nun gefunden, daß sich magnetische Partikel BioMag$^R$, welche an ihrer Oberfläche freie Aminogruppen tragen, durch Umsetzung mit 2-Iminothiolan (2-It) so aktivieren lassen, daß sie sich durch bloße Inkubation mit Antikörpern kovalent mit denselben verknüpfen lassen. Derartig hergestellte magnetische Antikörper-Konjugate sind neu.

Ferner wurde gefunden, daß sich magnetische Partikel BioMag$^R$, welche an ihrer Oberfläche freie Aminogruppen tragen, auch durch Umsetzung mit N-Succinimidyl-3-(2-pyridyldithio)-propionat (SPDP) und nachfolgender reduktiver Spaltung der Disulfidbindung mittels Dithiothreitol oder Mercaptoethanol so aktivieren lassen, daß sie sich durch bloße Inkubation mit Antikörpern kovalent mit denselben verknüpfen lassen. Derartig hergestellte magnetische Antikörper-Konjugate sind ebenfalls neu.

Weiterhin wurde gefunden, daß sich die genannten magnetischen Partikel nach Umsetzung mit SPDP auch durch Inkubation mit Antikörpern, welche freie SH-Gruppen tragen, kovalent mit den Antikörpern verknüpfen lassen, ohne daß die durch Umsetzung mit SPDP modifizierten magnetischen Partikel zuvor mittels Dithiothreitol oder Mercaptoethanol aktiviert werden müssen. Derartig hergestellte magnetische Antikörperkonjugate sind ebenfalls neu.

Überraschenderweise wurde gefunden, daß sich die Beladung der magnetischen Partikel mit Antikörper jeweils erhöhen läßt, wenn man etwaige nach dem Kopplungsschritt noch vorhandene Thiolgruppen durch Reaktion mit N-Ethyl-maleimid oder Jodacetamid absättigt. Überraschenderweise wird dadurch auch die Stabilität der hergestellten magnetischen Antikörper-Konjugate erhöht.

Insbesondere wurde gefunden, daß sich die jeweils hergestellte Disulfidbindung zwischen magnetischem Partikel und Antikörper durch Reduktion mit Dithiothreitol oder Mercaptoethanol wieder spalten läßt. Derartige spaltbare magnetische Partikel-Antikörper-Konjugate sind ebenfalls neu-und in der Spaltbarkeit des Spacers und der variablen Spacerlänge liegen zwei besondere Vorteile der Erfindung:

1. Der spaltbare Spacer ermöglicht eine Positivselektion derjenigen Antigene oder Zellen, die von dem an die magnetischen Partikel gekoppelten Antikörper erkannt werden - und zwar durch einfache magnetische Trennung. Die magnetischen Partikel lassen sich von den depletierten Antigenen oder Zellen auf reduktivem Wege wieder abspalten und mit Hilfe eines Magneten entfernen.

2. Die variable Spacerlänge erlaubt es, den Abstand zwischen Antikörper und magnetischem Partikel in gewissen Grenzen zu variieren und dem jeweiligen Kopplungs- bzw. Trenn- bzw. Depletionsproblem anzupassen. Diese Auswahlmöglichkeit ist vor allem bei Verwendung von Partikeln verschiedener Größe oder bei der Kopplung von Antikörpern verschiedener Klassen oder Isotypen von besonderem Vorteil.

Überraschenderweise wurde gefunden, daß die Spezifität und Reaktivität der über Disulfid-Bindungen bzw. über die beschriebenen Spacer an magnetische Partikel gekoppelte Antikörper voll erhalten bleibt, weil durch die Kopplung des Antikörpers über seine Hinge-Region seine Antigen-Bindungsstelle nicht verändert oder beeinträchtigt wird. Hierin liegt ein besonderer Vorteil der Erfindung gegenüber den bislang bekannten Kopplungsverfahren, bei denen die Antikörper, wie vorstehend beschrieben, entweder rein adsorptiv oder über Reaktion ihrer Aminogruppen auf magnetische Partikel aufgezogen werden, was sowohl die Spezifität als auch die Reaktivität der konjugierten Antikörper beeinträchtigen kann. Gegenüber der adsorptiven Kopplung besteht überdies bei der vorliegenden Erfindung der Vorteil, daß die Antikörper an die magnetischen Partikel chemisch gebunden sind.

Es wurde weiterhin gefunden, daß sich die erfindungsgemäßen magnetischen Antikörper-Konjugate, z. B. bei der Depletion von Knochenmark, wegen ihrer hohen Spezifität als besonders vorteilhaft erweisen.

Weiterhin wurde gefunden, daß sich die erfindungsgemäßen magnetischen Antikörper-Konjugate auch im Rahmen eines diagnostischen Verfahrens bzw. als Diagnostikum, insbesondere z. B. bei der HLA-Typisierung, wegen ihrer hohen Spezifität als vorteilhaft erweisen.

Insbesondere wurde gefunden, daß sich die erfindungsgemäßen magnetischen Antikörper-Konjugate zur Positiv-Selektion von Antigenen oder Zellen eignen, weil sich die magnetischen Partikel nach reduktiver Spaltung der Disulfidbindung des Spacers von den isolierten Antigenen oder Zellen mit Hilfe eines Magneten oder zentrifugativ wieder abtrennen lassen.

Die Herstellung erfindungsgemäßer magnetischer Antikörper-Konjugate ist nachfolgend für verschiedene monoklonale Antikörper, welche gegen Zellen des Knochenmarks gerichtet sind sowie für ein polyclonales Immunglobulin vom Kaninchen beispielhaft beschrieben; jedoch engen die genannten Beispiele die Erfindung nicht ein. Weiterhin ist die Verwendung der beispielhaft hergestellten magnetischen Antikörper-Konjugate zur Depletion von Zellen des Knochenmarks ebenfalls beispielhaft beschrieben, ohne die Verwendung auf die genannten Beispiele zu beschränken. Beispielhaft beschrieben ist auch die reduktive

Spaltung der Disulfidbindung des Spacers durch Inkubation mit Dithiothreitol, ohne die Erfindung dadurch einzuschränken.

Verfahren zur Herstellung magnetischer Protein-Konjugate der Formel I

a) Aminogruppen tragende magnetische Partikel M werden in einem geeigneten Lösungsmittel mit einer mit Aminogruppen reagierenden Verbindung der Formel II,

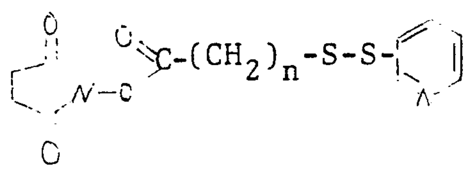

II

worin n = 1-6 ist, unter Ausbildung einer Amidbindung zu einer Verbindung der Formel III

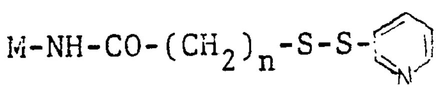

III

und diese durch reduktive Spaltung der Disulfidbindung zu einer Verbindung der Formel IV
$M\text{-}NH\text{-}CO\text{-}(CH_2)_n\text{-}SH$ IV
umgesetzt, welche schließlich in einem geeigneten wäßrigen salzhaltigen Lösungsmittel, welches Proteine nicht denaturiert, wie z. B. physiologische Kochsalzlösung oder eine mit Phosphat gepufferte Kochsalzlösung, mit einem Disulfidbindungen besitzenden Protein P, etwa einem Antikörper, zu einer Verbindung der Formel I umgesetzt wird, oder

b) Aminogruppen tragende Partikel M werden in einem geeigneten Lösungsmittel mit Iminothiolan zu einer Verbindung der Formel IV umgesetzt, in welcher n = 3 ist, worauf die Umsetzung dieser Verbindung der Formel IV mit einem Disulfidbindungen besitzenden Protein P nach der vorstehend beschriebenen Weise erfolgt, oder

c) Aminogruppen tragende Partikel M werden wie vorstehend beschrieben zu einer Verbindung der Formel III umgesetzt, welche in einem geeigneten wäßrigen salzhaltigen Lösungsmittel, welches Proteine nicht denaturiert, mit einem Thiolgruppen tragenden Protein P, etwa einem reduzierten Antikörper oder einem Fab- oder Fab′-Fragment, zu einer Verbindung der Formel I umgesetzt wird, worauf die Bindung zwischen Protein P und gespacertem magnetischen Partikel gegebenenfalls durch Zusatz eines geeigneten Maleimidoderivats, z. B. von N-Ethyl-maleimid, oder durch Zusatz von Jodacetamid oder Bromacetamid stabilisiert wird.

Geeignete Lösungsmittel zur Kopplung einer Verbindung der Formel II oder von Iminothiolan an magnetische Partikel müssen so beschaffen sein, daß die jeweils zur Kopplung verwendeten magnetischen Partikel in ihren physikalischen und magnetischen Eigenschaften, insbesondere in ihrer Größe, Dispergierbarkeit und Oberflächenbeschaffenheit, durch das verwendete Lösungsmittel nicht beeinträchtigt werden. Für magnetische Partikel, wie sie z. B. in EP 0125995 A2 oder WO 8303920 beschrieben sind, wurde als geeignetes Lösungsmittel z. B. eine Mischung aus Wasser und Dimethylformamid aufgefunden.

Bestimmung des Kopplungsgrades (µg Antikörper/mg Eisen)

Eisen wurde durch Atomabsorption und Stickstoff nach der Kjeldahl-Methode bestimmt. Die Werte für den gekoppelten Protein-Stickstoff wurden nach der Formel

$$\frac{\mu g\ P\text{-}N}{mg\ Fe} = \frac{\mu g\ Tot\text{-}N}{mg\ Fe}\ (Probe) - \frac{\mu g\ Tot\text{-}N}{mg\ Fe}\ (Kontrolle)$$

berechnet, wobei die Begriffe folgende Bedeutung haben:
P-N: Protein-Stickstoff

Tot-N.: Gesamt-Stickstoff
Fe: Eisen

Die an die Partikel gebundene Proteinmenge (µg Protein/mg Eisen) wurde aus der Proteinstickstoffmenge je mg Eisen durch Multiplikation mit dem Faktor 6.25 errechnet. Die errechneten Kopplungsraten sind in Tabelle 1 zusammengefaßt.

Verfahren zur Depletion von Zellen

Eine Suspension eines zu depletierenden Zellgemischs in einer salzhaltigen, bevorzugt physiologischen wäßrigen Lösung oder in einer Körperflüssigkeit wird mit einer Verbindung der Formel I bei einer geeigneten Temperatur zwischen z. B. 0° C und 40° C, bevorzugt unter Schütteln, ebenfalls bevorzugt unter sterilen Bedingungen, über einen geeigneten Zeitraum inkubiert, und danach werden die magnetischen Partikel durch einen geeigneten Magneten aus der Lösung abgetrennt.

Geeignete Temperaturen sind beispielsweise 0° C, Raumtemperatur oder 37° C, bevorzugt jedoch Raumtemperatur. Die Zeitdauer der Inkubation richtet sich jeweils nach der verwendeten Inkubationstemperatur und nach der Bindungsreaktivität des Antikörpers und kann z. B. von wenigen Minuten bis zu z. B. 2 Stunden betragen. Bevorzugterweise wird z. B. bei Raumtemperatur z. B. über einen Zeitraum von 10 bis 20 Minuten inkubiert.

Verfahren zur Isolierung löslicher bioorganischer Moleküle

Dieses Verfahren folgt im wesentlichen dem Verfahren zur Depletion von Zellen.

Beispiele

Die nachfolgenden Beispiele dienen der näheren Erläuterung der Erfindung, schränken die Erfindung jedoch nicht ein.

Magnetische Partikel, welche in der beschriebenen Weise mit monoklonalen Antikörpern zur Reaktion gebracht wurden, werden nachfolgend als "Magnetobeads" bezeichnet, wobei ihre Spezifität jeweils durch Voranstellung der jeweiligen Antikörper-Bezeichnung wiedergegeben wird.

Beispiel 1

Herstellung einer Verbindung der Formel IV, in welcher n = 3 ist

3 x 300 µl einer käuflichen Suspension der magnetischen Partikel (BioMag$^R$, Advanced Magnetics) wurden jeweils 3x mit je 10 ml Phosphat-gepufferter Kochsalzlösung pH 7.2 (PBS) gewaschen und jeweils in 2 ml PBS resuspendiert. Danach erfolgte jeweils die Zugabe einer Lösung von 2-Iminothiolan-Hydrochlorid (2-It HCl) in PBS:

a) Zusatz von 10 mg 2-It HCl in 0,5 ml PBS
b) Zusatz von 2.5 mg 2-It HCl in 0,5 ml PBS
c) Zusatz von 0.6 mg 2-It HCl in 0,5 ml PBS

Diese Suspensionen wurden jeweils 1 h bei Raumtemperatur geschüttelt. Dann wurden die Partikel bei 3000 x g abzentrifugiert und jeweils 3x mit je 10 ml PBS gewaschen.

Beispiel 2

Herstellung einer Verbindung der Formel III, in welcher n = 2 ist

3 x 300 µl einer käuflichen Suspension der magnetischen Partikel (BioMag$^R$, Advanced Magnetics) wurden jeweils 3x mit je 10 ml PBS pH 7.2 gewaschen und jeweils in 3 ml PBS resuspendiert. Dann wurde jeweils eine Lösung von 5 mg N-Succinimidyl-3-(2-pyridyldithio)-propionat (SPDP) in 2 ml trockenem Dimethylformamid (DMF) zugesetzt und 1 h bei Raumtemperatur geschüttelt. Danach wurden die Partikel bei 3000 x g abzentrifugiert und jeweils 3x mit je 10 ml PBS gewaschen.

Beispiel 3

Herstellung einer Verbindung der Formel IV, in welcher n = 2 ist

Drei gemäß Beispiel 2 hergestellte Partikel-Aliquote der Formel III wurden jeweils in 2.4 ml PBS resuspendiert, jeweils mit 5 mg Dithiothreitol, gelöst in jeweils 0.1 ml PBS, versetzt und 30 min bei Raumtemperatur unter leichtem Schütteln inkubiert. Dann wurden die Partikel bei 3000 x g abzentrifugiert, jeweils 3x mit je 10 ml PBS gewaschen und unverzüglich für die Kopplungsreaktionen verwendet.

Beispiel 4

Kopplung von polyklonalem Kaninchen-anti-Maus-Immunglobulin (KAM) an eine Verbindung der Formel IV gemäß Beispiel 1a, in welcher n = 3 ist, unter Variation der Antikörper-Menge

Vier gemäß Beispiel 1a aus jeweils 300 µl-Aliquoten BioMag$^R$ hergestellten Partikel-Aliquote der Formel IV wurden in

a) 2.5 ml PBS
b) 2.4 ml PBS
c) 2.3 ml PBS
d) 2.2 ml PBS resuspendiert und die Suspension mit folgenden Mengen KAM versetzt:
a) kein Zusatz
b) 0.5 mg KAM in 0.1 ml PBS
c) 1.0 mg KAM in 0.2 ml PBS
d) 1.5 mg KAM in 0.3 ml PBS

Die Mischungen (jeweils 2.5 ml) wurden jeweils 1 h unter Schütteln bei Raumtemperatur inkubiert. Dann wurden die Partikel bei 3000 x g abzentrifugiert, 3x mit je 10 ml PBS gewaschen, in 5 ml PBS pH 7.2 resuspendiert, mittels Röntgenbestrahlung sterilisiert und bei 4° C aufbewahrt. Die analytischen Daten sind in Tabelle 1 zusammengefaßt.

Beispiel 5

Kopplung des monoklonalen Antikörpers BMA 0110 (anti-CD2; IgG2b) an eine Verbindung der Formel IV gemäß Beispiel 1a, in welcher n = 3 ist, unter Variation der Antikörper-Menge

Die Kopplung erfolgte in Analogie zu Beispiel 4, wobei die Partikel-Suspensionen a-d der Formel IV mit folgenden Mengen BMA 0110 versetzt wurden:

a) kein Zusatz
b) 0.5 mg BMA 0110 in 0.1 ml PBS
c) 1.0 mg BMA 0110 in 0.2 ml PBS
d) 1.5 mg BMA 0110 in 0.3 ml PBS

Weiterverarbeitung erfolgte analog Beispiel 4; die analytischen Daten sind in Tabelle 1 aufgeführt.

Beispiel 6

Kopplung des monoklonalen Antikörpers BMA 0110 (anti-CD2; IgG2b) an eine Verbindung der Formel IV gemäß Beispiel 1b, in welcher n = 3 ist, unter Variation der Antikörper-Menge

Die Kopplung erfolgte in Analogie zu Beispiel 5, wobei jedoch von einer Verbindung der Formel IV gemäß Beispiel 1b (statt Beispiel 1a) ausgegangen wurde.

Beispiel 7

Kopplung des monoklonalen Antikörpers BMA 030 (anti-CD3; IgG2a) an eine Verbindung der Formel IV gemäß Beispiel 1a, 1b und 1c, in welcher n = 3 ist, bei Verwendung gleicher Antikörper-Konzentrationen

Gemäß Beispiel 1a, 1b und 1c hergestellte Partikel-Aliquote der Formel IV wurden jeweils in 2.0 ml PBS resuspendiert und jeweils mit 0.5 ml einer Lösung von BMA 030 in PBS (entsprechend jeweils 1 mg BMA 030 in 0.5 ml PBS) versetzt und analog Beispiel 4 weiterverarbeitet. Die analytischen Daten sind in Tabelle 1 zusammengefaßt.

Beispiel 8

Kopplung des monoklonalen Antikörpers BMA 033 (anti-CD3; IgG3) an eine Verbindung der Formel IV gemäß Beispiel 1a, 1b und 1c, in welcher n = 3 ist, bei Verwendung gleicher Antikörper-Konzentrationen

Die Umsetzung erfolgte in Analogie zu Beispiel 7; die analytischen Daten sind in Tabelle 1 aufgeführt.

Beispiel 9

Kopplung des monoklonalen Antikörpers BMA 081 (anti-CD8; IgG2a) an eine Verbindung der Formel IV gemäß Beispiel 1a, in welcher n = 3 ist, unter Variation der Antikörper-Menge

Die Umsetzung erfolgte in Analogie zu Beispiel 5; die analytischen Daten sind in Tabelle 1 aufgeführt.

Beispiel 10

Kopplung von polyklonalem Kaninchen-anti-Maus-Immunglobulin (KAM) an eine Verbindung der Formel III gemäß Beispiel 2, in welcher n = 2 ist, unter Variation der Antikörpermenge

Eine Lösung von 2 mg KAM in 0.4 ml PBS wurde mit 2 mg Dithiothreitol versetzt und 30 min bei Raumtemperatur inkubiert. Der reduzierte Antikörper wurde mittels Gelfiltration über Sephadex G25 in PBS pH 7.2 in einem Elutionsvoluiien von 4.2 ml isoliert.

Gemäß Beispiel 2 hergestellte Partikel-Aliquote der Formel III wurden wie folgt resuspendiert:

a) in 4.3 ml PBS
b) in 3.6 ml PBS
c) in 2.9 ml PBS

Diese Suspensionen wurden mit dem reduzierten Antikörper wie folgt versetzt:

a) Zusatz von 0.7 ml (ca. 0.3 mg Protein)
b) Zusatz von 1.4 ml (ca. 0.6 mg Protein)
c) Zusatz von 2.1 ml (ca. 0.9 mg Protein)

Die Mischungen (jeweils 5 ml) wurden jeweils 1 h unter Schütteln bei Raumtemperatur inkubiert. Dann wurden die Partikel bei 3000 x g abzentrifugiert, 3x mit je 10 ml PBS gewaschen, in 5 ml PBS pH 7.2 resuspendiert, mittels Röntgenstrahlung sterilisiert und bei 4 °C aufbewahrt. Die analytischen Daten sind in Tabelle 1 zusammengefaßt.

Beispiel 11

Kopplung von polyklonalem Kaninchen-anti-Maus-Immunglobulin (KAM) an eine Verbindung der Formel IV gemäß Beispiel 3, in welcher n = 2 ist, unter Variation der Antikörpermenge

Vier gemäß Beispiel 3 hergestellte Partikel-Aliquote der Formel IV wurden in:

a) 2.5 ml PBS
b) 2.4 ml PBS
c) 2.3 ml PBS
d) 2.2 ml PBS resuspendiert und die Suspension mit folgenden Mengen KAM versetzt:
a) kein Zusatz
b) 0.5 mg in 0.1 ml PBS
c) 1.0 mg in 0.2 ml PBS
d) 1.5 ml in 0.3 ml PBS

Die Mischungen (jeweils 2.5 ml) wurden jeweils 1 h unter Schütteln bei Raumtemperatur inkubiert. Dann wurden die Partikel bei 3000 x g abzentrifugiert, 3x mit je 10 ml PBS gewaschen, in 5 ml PBS pH 7.2 resuspendiert, mittels Röntgenbestrahlung sterilisiert und bei 4 °C aufbewahrt. Die analytischen Daten sind in Tabelle 1 zusammengefaßt.

Beispiel 12

Kopplung des monoklonalen Antikörpers BMA 0110 (anti-CD2; IgG2b) an eine Verbindung der Formel IV gemäß Beispiel 3, in welcher n = 2 ist, unter Variation der Antikörpermenge

Die Kopplung erfolgte in Analogie zu Beispiel 11; die analytischen Daten sind in Tabelle 1 aufgeführt.

Tabelle 1

| Kopplungsgrade verschieden hergestellter Magnetobeads | | | | | | | |
|---|---|---|---|---|---|---|---|
| Beispiel | Antikörper | Isotyp | Spezifität | Kopplungsmethode | Kopplungsansatz | | |
| | | | | | Ak/Fe 1) (mg/mg) | Sp/Fe 2) (μmol/mg) | P/Fe 3) (μg/mg) |
| 4b | KAM | IgG | aMIg | 2-It/SS | 0.1 | 14.5 | 110 |
| 4c | KAM | IgG | aMIg | 2-It/SS | 0.2 | 14.5 | 173 |
| 4d | KAM | IgG | aMIg | 2-It/SS | 0.3 | 14.5 | 229 |
| 5b | BMA 0110 | IgG2b | CD2 | 2-It/SS | 0.1 | 14.5 | 107 |
| 5c | BMA 0110 | IgG2b | CD2 | 2-It/SS | 0.2 | 14.5 | 156 |
| 5d | BMA 0110 | IgG2b | CD2 | 2-It/SS | 0.3 | 14.5 | 178 |
| 6b | BMA 0110 | IgG2b | CD2 | 2-It/SS | 0.1 | 3.6 | 65 |
| 6c | BMA 0110 | IgG2b | CD2 | 2-It/SS | 0.2 | 3.6 | 78 |
| 6d | BMA 0110 | IgG2b | CD2 | 2-It/SS | 0.3 | 3.6 | 112 |
| 7a | BMA 030 | IgG2a | CD3 | 2-It/SS | 0.2 | 14.5 | 71 |
| 7b | BMA 030 | IgG2a | CD3 | 2-It/SS | 0.2 | 3.6 | 53 |
| 7c | BMA 030 | IgG2a | CD3 | 2-It/SS | 0.2 | 0.9 | 12 |
| 8a | BMA 033 | IgG3 | CD3 | 2-It/SS | 0.2 | 14.5 | 69 |
| 8b | BMA 033 | IgG3 | CD3 | 2-It/SS | 0.2 | 3.6 | 56 |
| 8c | BMA 033 | IgG3 | CD3 | 2-It/SS | 0.2 | 0.9 | 39 |
| 9b | BMA 081 | IgG2a | CD8 | 2-It/SS | 0.1 | 14.5 | 73 |
| 9c | BMA 081 | IgG2a | CD8 | 2-It/SS | 0.2 | 14.5 | 80 |
| 9d | BMA 081 | IgG2a | CD8 | 2-It/SS | 0.3 | 14.5 | 91 |
| 10a | KAM | IgG | aMIg | SPDP/SH | 0.06 | 3.2 | 29 |
| 10b | KAM | IgG | aMIg | SPDP/SH | 0.12 | 3.2 | 36 |
| 10c | KAM | IgG | aMIg | SPDP/SH | 0.18 | 3.2 | 55 |
| 11b | KAM | IgG | aMIg | SPDP/red | 0.1 | 3.2 | 27 |
| 11c | KAM | IgG | aMIg | SPDP/red | 0.2 | 3.2 | 51 |
| 11d | KAM | IgG | aMIg | SPDP/red | 0.3 | 3.2 | 74 |
| 12b | BMA 0110 | IgG2b | CD2 | SPDP/red | 0.1 | 3.2 | 27 |
| 12c | BMA 0110 | IgG2b | CD2 | SPDP/red | 0.2 | 3.2 | 47 |
| 12d | BMA 0110 | IgG2b | CD2 | SPDP/red | 0.3 | 3.2 | 57 |

1) für den jeweiligen Kopplungsansatz verwendete Menge Antikörper je Eisen (mg/mg)
2) für den jeweiligen Kopplungsansatz verwendete Menge Spacer je Eisen (μmol/mg)
3) gekoppelte Proteinmenge je Eisen (μg/mg)
KAM: Kaninchen-anti-Maus-Immunglobulin
CD: Cluster of differentiation
aMIg: anti-Maus-Immunglobulin

**Ansprüche**

1. Magnetisches Protein-Konjugat der Formel I,

$M\text{-}NH\text{-}CO\text{-}(CH_2)_n\text{-}S\text{-}S\text{-}P$ I

mit n = 1-6, bevorzugt mit n = 2 oder 3, worin M ein dispergierbares, magnetisch reagierendes Material oder Partikel ist, welches Aminogruppen trägt, und P ein Protein ist.

2. Magnetisches Protein-Konjugat nach Anspruch 1,

**dadurch gekennzeichnet,**

daß die Thiolgruppe oder Thiolgruppen des Proteins P entweder in natürlicher Weise vorhanden sind oder durch Reduktion von Disulfidbindungen erzeugt oder durch chemische Reaktion in das Protein eingeführt

werden.

3. Magnetisches Protein-Konjugat nach Anspruch 1,
**dadurch gekennzeichnet,**
daß P ein polyklonales Immunglobulin ist.

4. Magnetisches Protein-Konjugat nach Anspruch 1,
**dadurch gekennzeichnet,**
daß P ein monoklonaler Antikörper oder ein Fab-, Fab′- oder $F(ab')_2$-Fragment ist.

5. Magnetisches Protein-Konjugat nach Anspruch 1,
**dadurch gekennzeichnet,**
daß P ein Antigen oder ein Enzym-, Hormon-, Lectin-oder Wachstumsfaktorrest ist.

6. Magnetisches Protein-Konjugat nach Anspruch 4,
**dadurch gekennzeichnet,**
daß P einen monoklonalen Antikörper der IgG- oder IgM-Klasse darstellt.

7. Magnetisches Protein-Konjugat nach Anspruch 4,
**dadurch gekennzeichnet,**
daß P einen monoklonalen Antikörper darstellt, welcher gegen ein Antigen gerichtet ist, welches in wäßrigen Salzlösungen oder Körperflüssigkeiten in gelöster Form vorhanden ist.

8. Magnetisches Protein-Konjugat nach Anspruch 4,
**dadurch gekennzeichnet,**
daß P einen monoklonalen Antikörper darstellt, welcher gegen ein Antigen gerichtet ist, welches auf Zellen, insbesondere auf Zellen des myeloischen oder lymphatischen Systems oder des peripheren Bluts, insbesondere auf B-Lymphozyten, T-Lymphozyten oder deren Vorläuferzellen oder auf Tumorzellen, insbesondere auf Tumorzellen des Knochenmarks, exprimiert ist.

9. Magnetisches Protein-Konjugat nach Anspruch 4,
**dadurch gekennzeichnet,**
daß P einen monoklonalen Antikörper darstellt, welcher gegen ein Antigen gerichtet ist, welches auf Bakterien, Mykoplasmen oder Protozoen oder aber auf Viren exprimiert ist.

10. Magnetisches Protein-Konjugat nach Anspruch 1,
**dadurch gekennzeichnet,**
daß P ein Antigen darstellt.

11 Magnetisches Protein-Konjugat nach Anspruch 1,
**dadurch gekennzeichnet,**
daß M ein dispergierbares Partikel mit Metalloxid-Core und einem Aminogruppen tragenden Hüllmantel ist, wobei das Metalloxid-Core eine Gruppe paramagnetischer Substanzen einschließen kann.

12. Magnetisches Protein-Konjugat nach Anspruch 11,
**dadurch gekennzeichnet,**
daß der Durchmesser der Partikel zwischen etwa 0,1 µ und etwa 100 µ, bevorzugt jedoch zwischen etwa 0,1 µ und etwa 1,5 µ liegt.

13. Verbindung der Formel III

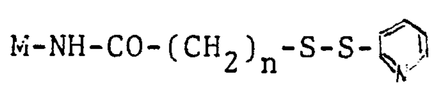

III

worin M die in Anspruch 1 angegebene Bedeutung hat.

14. Verbindung der Formel IV
$M\text{-}NH\text{-}CO\text{-}(CH_2)_n\text{-}SH$ IV
worin M die in Anspruch 1 angegebene Bedeutung hat.

15. Verfahren zur Herstellung eines magnetischen Proteinkonjugats der Formel I,
$M\text{-}NH\text{-}CO\text{-}(CH_2)_n\text{-}S\text{-}S\text{-}P$ I
**dadurch gekennzeichnet,**
daß man Aminogruppen tragende magnetische Partikel M mit einer mit Aminogruppen reagierenden Verbindung der Formel II,

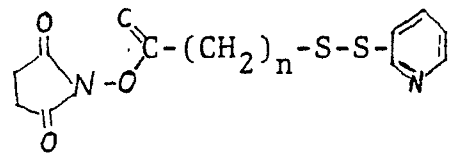

II

worin n = 1-6 ist, unter Ausbildung einer Amidbindung zu einer Verbindung der Formel III

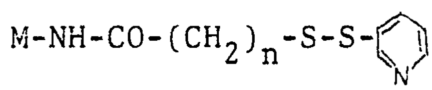

III

und diese durch reduktive Spaltung der Disulfidbindung zu einer Verbindung der Formel IV
M-NH-CO-(CH2)n-SH IV
umsetzt, welche schließlich mit einem Disulfidbindungen besitzenden Protein P zu einer Verbindung der Formel I umgesetzt wird.

16. Verfahren zur Herstellung eines magnetischen Protein-Konjugats der Formel I,
**dadurch gekennzeichnet,**
daß man Aminogruppen tragende magnetische Partikel M mit Iminothiolan zu einer Verbindung der Formel IV
$M-NH-CO-(CH_2)_n-SH$ IV
umsetzt, in welcher n = 3 ist, und die Verbindung der Formel IV mit einem Disulfidbindungen besitzenden Protein P zu einer Verbindung der Formel I umsetzt.

17. Verfahren zur Herstellung eines magnetischen Protein-Konjugats der Formel I,
**dadurch gekennzeichnet,**
daß man Aminogruppen tragende magnetische Partikel M wie in Anspruch 15 zu einer Verbindung der Formel III umsetzt und diese mit einem Thiolgruppen tragenden Protein P zu einer Verbindung der Formel I umsetzt.

18. Verfahren zur Herstellung eines magnetischen Protein-Konjugats nach mindestens einem der Ansprüche 15, 16 oder 17,
**dadurch gekennzeichnet,**
daß man die Bindung zwischen Protein P und dem gespacerten magnetischen Partikel durch Zusatz einer N-substituierten Maleimidoverbindung, bevorzugt durch Zusatz von N-($C_1$-$C_6$-Alkyl)-Maleimid, besonders bevorzugt durch Zusatz von N-Ethyl-Maleimid, oder durch Zusatz einer Maleimidocarbonsäure oder durch Zusatz von Jodacetamid oder Bromacetamid stabilisiert.

19. Verfahren zur Entfernung eines gelösten Antigens, Antikörpers, Rezeptors, Substrats, Co-Faktors oder einer Kohlenhydrat-Determinante aus wäßrigen Salzlösungen oder Körperflüssigkeiten,
**dadurch gekennzeichnet,**
daß man die Lösung mit einem geeigneten magnetischen Protein-Konjugat der Formel I inkubiert und das magnetische Protein-Konjugat nach spezifischer Adsorption der zu entfernenden Komponente auf magnetischem Wege abtrennt und die spezifisch adsorbierte Komponente gegebenenfalls wieder vom magnetischen Protein-Konjugat abeluiert oder durch Reduktion der Disulfidbindung des Spacers der Verbindung der Formel I abspaltet.

20. Verfahren zur Entfernung von Zellen aus wäßrigen Salzlösungen oder Körperflüssigkeiten,
**dadurch gekennzeichnet,**
daß man die Zellsuspension mit einem geeigneten magnetischen Protein-Konjugat der Formel I inkubiert und das magnetische Protein-Konjugat nach spezifischer Adsorption der zu entfernenden Zellen auf magnetischem Wege abtrennt und die spezifisch adsorbierten Zellen oder Partikel gegebenenfalls wieder vom magnetischen Protein-Konjugat ablöst oder durch Reduktion der Disulfidbindung des Spacers der Verbindung der Formel I abspaltet.

21. Verfahren zur Positiv-Selektion löslicher Komponenten gemäß Anspruch 19 oder von Zellen gemäß Anspruch 20,
**dadurch gekennzeichnet,**
daß man die Disulfidbindung des Spacers reduktiv spaltet und die magnetischen Partikel mittels eines Magneten oder zentrifugativ entfernt.

22. Verwendung eines magnetischen Protein-Konjugats nach Anspruch 1 zur spezifischen Entfernung von Zellen oder löslichen Antigenen, Rezeptoren, Substraten, Co-Faktoren oder Kohlenhydrat-Determinanten aus wäßrigen Salzlösungen oder Körperflüssigkeiten oder Verwendung im Rahmen eines diagnostischen Verfahrens bzw. als Diagnostikum.

23. Verwendung eines magnetischen Protein-Konjugats nach Anspruch 1 zur Entfernung von Zellen gemäß Anspruch 8 oder 9, bevorzugt zur Knochenmarks-Depletion, oder zur HLA-Typisierung.

24. Verwendung eines magnetischen Protein-Konjugats nach Anspruch 1 zur Positiv-Selektion von Zellen oder löslichen Antigenen, Rezeptoren, Substraten, Co-Faktoren oder Kohlenhydrat-Determinanten, insbesondere jedoch zur Positiv-Selektion von Zellen, insbesondere zur Positiv-Selektion von Zellen gemäß Anspruch 8.

Patentansprüche für folgende Vertragsstaaten: ES, GR

1. Verfahren zur Herstellung eines magnetischen Protein-Konjugats der Formel I,
$M-NH-CO-(CH_2)_nS-S-P$ I
dadurch gekennzeichnet, daß man Aminogruppen tragende magnetische Partikel M mit einer mit Aminogruppen reagierenden Verbindung der Formel II,

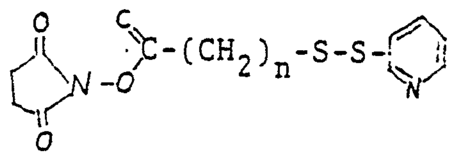

II

worin n = 1-6 ist, unter Ausbildung einer Amidbindung zu einer erbindung der allgemeinen Formel III

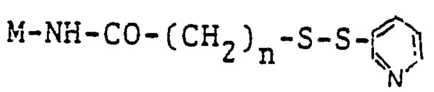

III

und diese durch reduktive Spaltung der Disulfidbindung zu einer Verbindung der Formel IV
$M-NH-CO-(CH_2)_n-SH$ IV
umsetzt, welche schließlicht mit einem Disulfidbindungen besitzenden Protein P zu einer Verbindung der Formel I umgesetzt wird.

2. Verfahren nach Anspruch 1, dadurch gekennzeichnet, daß das verwendete Protein eine Thiolgruppe trägt.

3. Verfahren nach Anspruch 1, dadurch gekennzeichnet, daß P ein polyklonales Immunglobulin ist.

4. Verfahren nach Anspruch 1, dadurch gekennzeichnet, daß P ein monoklonaler Antikörper, ein Fab-, Fab'-oder $F(ab')_2$-Fragment, ein Antigen oder ein Enzym-, Hormon-, Lectin- oder Wachstumsfaktorrest ist.

5. Verfahren nach Anspruch 1, dadurch gekennzeichnet, daß P einen monoklonalen Antikörper der IgG- oder IgM-Klasse darstellt.

6. Verfahren nach Anspruch 1, dadurch gekennzeichnet, daß M ein dispergierbares Partikel mit Metalloxid-Core und einem Aminogruppen tragenden Hüllmantel ist.

7. Verfahren nach Anspruch 1, dadurch gekennzeichnet, daß M ein dispergierbares Partikel mit Metalloxid-Core und einem Aminogruppen tragenden Hüllmantel ist und der Durchmesser der Partikel zwischen etwa 0, 1 µ und etwa 100 µ liegt.